(19) Europäisches Patentamt
European Patent Office
Office européen des brevets

(11) EP 0 492 401 B1

(12) **EUROPÄISCHE PATENTSCHRIFT**

(45) Veröffentlichungstag und Bekanntmachung des Hinweises auf die Patenterteilung:
**24.01.1996 Patentblatt 1996/04**

(51) Int. Cl.$^6$: **C07C 33/20**, C07C 29/17, C07D 211/14

(21) Anmeldenummer: **91121704.0**

(22) Anmeldetag: **18.12.1991**

# (54) Asymmetrische Hydrierung

Asymmetric hydrogenation

Hydrogénation asymmétrique

(84) Benannte Vertragsstaaten:
**AT BE CH DE DK ES FR GB GR IT LI LU NL SE**

(30) Priorität: **21.12.1990 CH 4100/90**

(43) Veröffentlichungstag der Anmeldung:
**01.07.1992 Patentblatt 1992/27**

(73) Patentinhaber: **CIBA-GEIGY AG**
**CH-4002 Basel (CH)**

(72) Erfinder:
- **Broger, Emil Albin, Dr.**
  **CH-4312 Magden (CH)**
- **Crameri, Yvo, Dr.**
  **CH-4104 Oberwil (CH)**
- **Isenring, Hans Peter, Dr.**
  **CH-4450 Sissach (CH)**
- **Pfiffner, Albert, Dr.**
  **CH-8180 Bülach (CH)**

(74) Vertreter: **Zumstein, Fritz, Dr. et al**
**D-80331 München (DE)**

(56) Entgegenhaltungen:
**EP-A- 0 032 659** **DE-A- 2 752 096**
- **CHEMISTRY LETTERS, The Chemical Society of Japan, Nr. 7, 1985, Tokyo, JP Seiten 1007 - 1008; S. INOUE et al: "Asymmetric hydrogenation of geraniol and nerol catalyzed by binap-rhodium(I) complexes"**
- **M. Nográdi, Stereoselective Synthesis, Weinheim, 1987, S. 58 - 74.**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 492 401 B1

Printed by Rank Xerox (UK) Business Services
2.9.7/3.4

## Beschreibung

Die vorliegende Erfindung betrifft eine asymmetrische Hydrierung zur Herstellung von Verbindungen der Formel

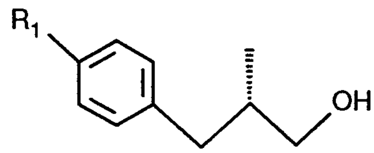

I

worin $R_1$ Wasserstoff oder eine Gruppe Z,
Z die Gruppe

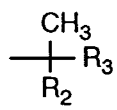

,

$R_2$ Methyl, Ethyl, Chlormethyl und
$R_3$ $C_1$-$C_4$-Alkyl bedeuten,
oder $R_2$ und $R_3$ zusammen mit dem Kohlenstoffatom, an das sie gemeinsam gebunden sind, $C_3$-$C_7$-Cycloalkyl darstellen.

Das Verfahren ist dadurch gekennzeichnet, dass man eine Verbindung der Formel

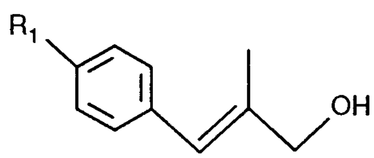

II

in der E-Form,
worin $R_1$ obige Bedeutung hat, asymmetrisch hydriert in Gegenwart eines Rhodiumkatalysators der Formeln V oder VI

$$[Rh(X)(Y)L_{0,1,2}]_{1,2} \qquad V$$

oder

$$[Rh(Y)L_{0,1,2}]^{+}A^{-} \qquad VI$$

worin bedeuten:
X ein koordinierender anionischer Ligand, wie Halogen, ein Carbonsäurerest, ein 1,3-Diketonat, ein (gegebenenfalls substituiertes) Phenolat, Hydroxy, Nitrat, Nitrit, Cyanat, Rhodanid, Cyanid, Hydrogensulfat;

Y ein chiraler atropisomerer Diphosphinligand, der Formeln VII oder VIII:

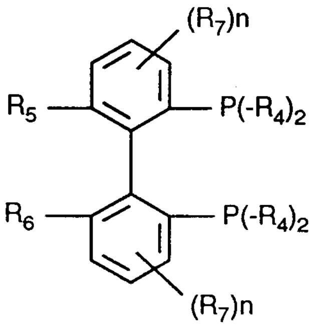

VII

worin $R_4$ Aryl, Cyclohexyl, $R_5$ und $R_6$ Wasserstoff, niederes Alkyl, niederes Alkoxy, Di-niederes Alkylamino, geschütztes Hydroxymethyl oder $R_5$ und $R_6$ zusammen die Gruppen $(-CH_2)-)_m$, $-CH_2-O-CH_2-$, $-CH_2-NR_8-CH_2-$ oder $-CH_2-C(OR_9)_2-CH_2-$ bedeuten, wobei m eine Zahl 3 bis 5, $R_8$ niederes Alkyl, Aryl oder Benzyl und $R_9$ niederes Alkyl oder beide $R_9$ zusammen Di- oder Trimethylen darstellen, $R_7$ Methyl, niederes Alkoxy, Di-niederes Alkylamino oder Halogen und n die Zahl 0, 1, 2 oder 3 bedeuten;

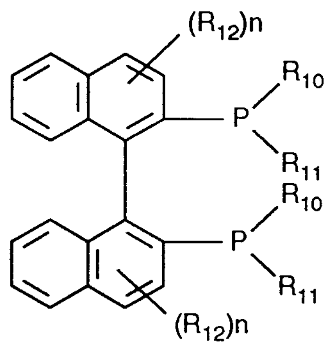

VIII

worin $R_{10}$ und $R_{11}$ Aryl und Cyclohexyl, $R_{12}$ Methyl, Ethyl, Halogen, -OH; $NH_2$, Acetylamino, Nitro, $-SO_3H$, bevorzugt in der 5,5'-Position,

L ein neutraler Ligand,

A- ein Anion, insbesondere $BF_4$- bedeuten.

VII und VIII werden erfindungsgemäss in der (R)-Form eingesetzt.

Verbindungen der Formel I sind wertvolle Zwischenprodukte zur Herstellung von optisch aktiven Fungiziden, die in Form ihrer Racemate z.B. aus DE-A-27,52,096 bekannt sind.

Aus EP-A-32,659 ist bekannt, dass optisch aktive Phenylpropanole durch mikrobiologische Hydrierung aus den entsprechend ungesättigten Phenylpropenolen hergestellt werden können. Ferner ist aus Chemistry Letters 1007-8 (1985) und DE-A-30,18,388 bekannt, dass substituierte Allylalkohole in Gegenwart optisch aktiver Phosphin-Rhodium-Katalysatoren zu den entsprechenden optisch aktiven gesättigten Alkoholen hydriert werden können. Die darin beschriebenen Verbindungen unterscheiden sich aber strukturell von denjenigen der vorliegenden Erfindung; zudem sind die optischen Ausbeuten und die erforderliche Menge Katalysator unbefriedigend für eine technische Anwendung.

Das erfindungsgemässe Verfahren ermöglicht die Herstellung von optisch aktiven Phenylpropanolen in hohen chemischen und optischen Ausbeuten unter Verwendung von geringen Mengen an Katalysator und bei kurzen Reaktionszeiten.

Der Ausdruck Halogen bedeutet im Rahmen der vorliegenden Erfindung Fluor, Chlor, Brom oder Jod.

Bevorzugte Liganden sind diejenigen der Formel VII. Von diesen wiederum sind diejenigen bevorzugt, worin $R^4$ unsubstituiertes oder Methyl-substituiertes Phenyl, $R^5$ und $R^6$ gleich sind und niederes Alkyl, Alkoxy oder zusammen die Gruppe $-CH_2-O-CH_2-$, n die Zahl 0 oder 1 und $R^7$ Methyl, Fluor oder Di-niederes Alkylamino bedeuten. Falls n die Zahl 1 bedeutet, befindet sich der Substituent $R^7$ vorzugsweise in meta-Stellung zum Phosphoratom.

Als Beispiele von besonders bevorzugten Liganden der Formel VII können die folgenden genannt werden:

R-(6,6'-Dimethyl-2,2'-biphenylylen)bis(diphenylphosphin);
R-(6,6'-Dimethyl-2,2'-biphenylylen)bis(di-p-tolylphosphin);
R-(6,6'-Dimethoxy-2,2'-biphenylylen)bis(diphenylphosphin);
R-(6,6'-Dimethoxy-2,2'-biphenylylen)bis(di-p-tolylphosphin).

Die Liganden der Formeln VII und VIII sind bekannte Verbindungen, oder können in an sich bekannter Weise hergestellt werden.

Der Ausdruck "niederes Alkyl" allein oder als Teil eines Substituenten wie "niederes Alkoxy" oder "di-niederes Alkylamino" bedeutet im Rahmen der vorliegenden Erfindung geradkettige oder verzweigte Alkylgruppen mit 1 bis 9 Kohlenstoffatomen wie z.B. Methyl, Aethyl, Propyl, Isopropyl, n-Butyl; sek. Butyl, Isobutyl, tert. Butyl sowie auch Pentyl, Hexyl, Heptyl, Octyl, Nonyl und ihre Isomeren. Die Definition "$C_{1-4}$-Alkylreste" betrifft die ersten acht dieser Bedeutungen.

$C_3$-$C_7$-Cycloalkyl bedeutet Cyclopropyl, Cyclobutyl, Cyclopentyl, Cyclohexyl und Cycloheptyl

Auch die "$C_{1-4}$-Alkylreste" können geradkettig oder verzweigt sein.

Der im Zusammenhang mit den Verbindungen der Formel VII und VIII verwendete Ausdruck "Aryl" bedeutet im Rahmen der vorliegenden Erfindung Phenyl, welches gegebenenfalls in ortho-, para- und/oder meta-Stellung niedere Alkyl- oder niedere Alkoxygruppen, vorzugsweise Methyl- oder Methoxygruppen, oder auch di-niederes Alkylamino, vorzugsweise Dimethylaminogruppen aufweisen kann.

Der Ausdruck "niederes Alkoxy" bedeutet Gruppen. in denen der Alkylrest die vorhergehende Bedeutung hat. Weiterhin bedeutet das Zeichen "-·ıı ", dass sich der entsprechende Rest unterhalb der Molekülebene befindet.

Der Ausdruck "neutraler Ligand" bedeutet im Rahmen der vorliegenden Erfindung einen leicht austauschbaren Liganden wie Olefine, z.B. Aethylen, Propylen, Cycloocten, 1,5-Hexadien, Norbornadien, 1,5-Cyclooctadien und dergleichen, Nitrile wie Acetonitril, Benzonitril, oder auch das verwendete Lösungsmittel usw. Dieser Ligand kann bei der Hydrierung ausgetauscht werden. Falls mehr als ein solcher Ligand vorhanden ist, können diese auch voneinander verschieden sein.

Ueberraschenderweise wurde nun gefunden, dass die Rhodiumdiphosphinkomplexe der Formeln V und VI im Vergleich zu den vorbekannten Katalysatoren für solche Zwecke erheblich aktiver und enantioselektiver sind, was insbesondere dazu führt, dass durch ihre Verwendung erheblich geringere Katalysatormengen verwendet werden können, kürzere Reaktionszeiten möglich sind und optische Ausbeuten (e.e.) von über 95% erzielt werden können.

Die asymmetrischen Hydrierungen können in geeigneten, unter den Reaktionsbedingungen inerten organischen Lösungsmitteln durchgeführt werden. Als solche können insbesondere genannt werden aromatische Kohlenwasserstoffe wie Benzol oder Toluol, cyclische Aether wie Tetrahydrofuran oder Dioxan. Ester wie z.B. Essigester oder auch Gemische hiervon und dergleichen. Das Verhältnis zwischen Rhodium und dem Liganden Y liegt zweckmässig zwischen etwa 0,05 und etwa 5 Mol, vorzugsweise zwischen etwa 0,5 und etwa 2 Mol Rhodium pro Mol Ligand. Das Verhältnis zwischen Rhodium und dem Rest X liegt zwischen etwa 0,01 und etwa 20, vorzugsweise zwischen etwa 0,5 und etwa 10 Mol Rhodium pro Mol Rest X. Das molare Verhältnis zwischen Rhodium in den Komplexen der Formeln V und VI und den zu hydrierenden Verbindungen II liegt zweckmässig zwischen etwa 0,001 und etwa 5 Mol.%, vorzugsweise zwischen etwa 0,002 und etwa 0,02 Mol.%.

Die asymmetrischen Hydrierungen unter Verwendung der Komplexe der Formel V oder VI können zweckmässig bei Temperaturen von etwa 20°C bis etwa 140°C, vorzugsweise von etwa 80°C bis etwa 120°C, durchgeführt werden. Diese Hydrierungen erfolgen zweckmässig unter Druck, insbesondere unter einem Druck von etwa 1 bis 100 bar, vorzugsweise 2 bis 60 bar.

Die Verbindungen der Formel I sind Zwischenprodukte.

Diese dienen insbesondere der Herstellung der S-Isomeren der Verbindungen der Formel

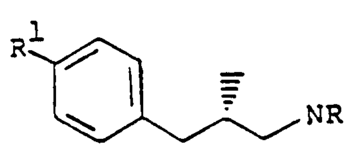

IV

mit NR = N-Piperidyl, 3,5-cis-Dimethylpiperidyl, 3-Methylpiperidyl, 2,6-Dimethyl-4-morpholinyl, in die sie in an sich bekannter Weise übergeführt werden können, z.B. gemäss

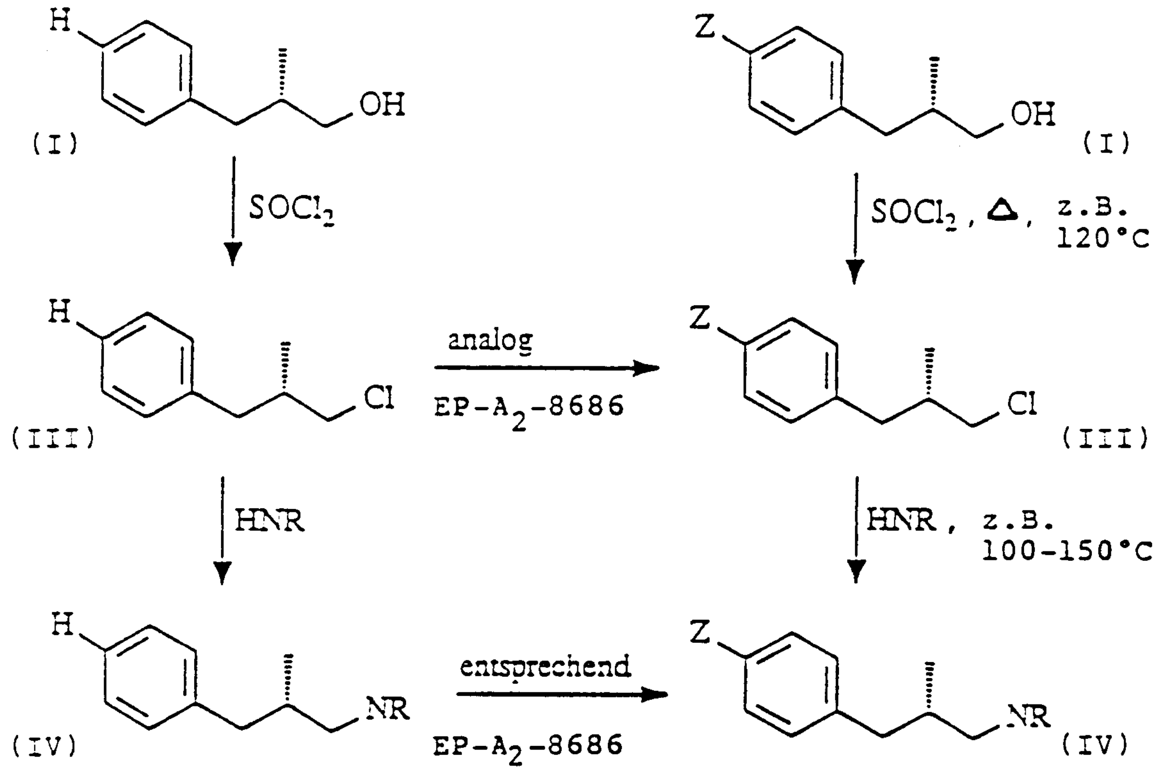


So wird also z.B. durch üblichen Austausch der Hydroxylgruppe von I durch Chlor, z.B. mittels $SOCl_2$ zunächst eine Verbindung

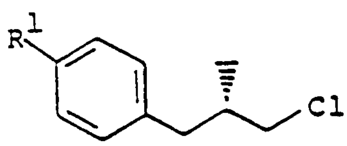

III

erhalten, welche letztere durch Behandlung mit einem Amin der Formel HNR in IV übergeführt werden kann.

Die Reaktion wird zweckmässigerweise bei erhöhter Temperatur durchgeführt, und das Amin - da Reagens und Lösungsmittel - zweckmässigerweise im Ueberschuss eingesetzt.

Aber auch der Weg gemäss EP-A2 8686 ist möglich, d.h. beinhaltend Alkylierung des Phenylrestes.

Die Hydroxygruppe in I muss dabei also wiederum zunächst so aktiviert werden, dass eine nukleophile Substitution durch ein Amin möglich wird. Die üblichen Gruppen sind demzufolge Halogenide (Cl, Br, J), aber auch Sulfonate (z.B.

Tosylat, Mesylat) sind zweckdienlich.

| Beispiele | |
|---|---|
| Abkürzungen | |
| (R)-BIPHEMP | (R)-(6,6'-Dimethyl-2,2'-biphenylylen)bis(diphenylphosphin) |
| (R)-pTolBINAP | [((R)-1,1'-Binaphthyl)-2,2'-ylen]bis(di-p-tolylphosphin) |
| (R)-pTolMeOBIPHEP | [(R)-6,6'-Dimethoxy-2,2'-biphenylylen]bis(di-p-tolylphosphin) |
| (R)-BIPHOMP | [(R)-5,7-Dihydro-dibenz[c,e]oxepin-1,11-diyl]bis(diphenylphosphin) |
| AcOEt | Essigsäureäthylester |
| THF | Tetrahydrofuran |
| EtOH | Aethanol |

Bestimmung der e.e.-Werte

Zur Bestimmung der e.e.-Werte werden die Produkte in Methylenchlorid mit (R)- oder (S)-6-Methoxy-2,5,7,8-tetramethylchroman-2-carbonsäure, Dicyclohexylcarbodiimid und 4-Dimethylaminopyridin in die diastereomeren Ester überführt und gaschromatographisch analysiert.

Beispiel 1

In einer Glove-Box ($O_2$-Gehalt <1 ppm) werden in einem 50 ml Messkolben 39,2 mg (0,049 mMol) Tetrabutylammoniumhydroxid 30-Hydrat, 7,5 mg (0,049 mMol) 2,6-Dihydroxybenzoesäure, 19,9 mg (0,049 mMol) Bis-(1,5-Cyclooctadien)rhodium(I)tetrafluoroborat und 33,2 mg (0,049 mMol) (R)-pTolBINAP in 50 ml Toluol suspendiert. Die Suspension wird anschliessend während 1,5 Stunden bei 22°C gerührt. Es bildet sich eine orange-rote, klare Katalysatorlösung.

Beispiel 2

In einer Glove-Box ($O_2$-Gehalt) < 1 ppm) wird ein 500 ml-Autoklav mit 5,0 g (24,5 mMol) (E)-Dehydroliliol, 145 ml Toluol und 5 ml nach Beispiel 1 hergestellter Katalysatorlösung beladen. Die Hydrierung wird bei 100°C, einem konstanten Druck von 60 bar $H_2$ und unter intensivem Rühren durchgeführt. Nach 6 Stunden beträgt der Umsatz > 99%. Die hellgelbe Hydrierlösung wird aus dem Autoklaven gespült und am Rotationsverdampfer bei 60°/17 mbar eingedampft. Der Rückstand wird bei 140°/0,01 mbar destilliert. Man erhält 5,0 g (99,0%) (S)-Liliol, als farbloses Oel, mit einer enantiomeren Reinheit von 92,4% e.e. $[\alpha]_{365}^{20}$ = -46,0° (EtOH, c = 1%).

Beispiele 2a-

In zu Beispiel 1 analoger Weise wird eine Katalysatorlösung hergestellt und anschliessend die Hydrierung von (E)-Dehydroliliol analog Beispiel 2 durchgeführt. Die Resultate sind in Tabelle 1 zusammengefasst:

Tabelle 1

| Beispiele 2a-2i | | | | | | | | | |
|---|---|---|---|---|---|---|---|---|---|
| No 2 | [Rh(X)(Y)(COD)] X | Y | S/C | Solvens | c % | p bar | T °C | % Umsatz nach 22 h | Liliol e.e. % |
| a | $CH_3OCH_2COO$ | (R)-pTolBINAP | 5'000 | Toluol | 3,7 | 60 | 100 | 99,2 | 93,7(S) |
| b | $Cl_2CHCOO$ | " | 5'000 | " | 3,7 | 60 | 100 | 99,4 | 95,3(S) |
| c | $C_6H_5COO$ | " | 5'000 | " | 3,7 | 60 | 100 | 100,0 | 94,6(S) |
| d | $C_6Cl_5OCH_2COO$ | " | 5'000 | " | 3,7 | 60 | 100 | 89,7 | 94,9(S) |
| e | $(CH_3)_3CCOO$ | " | 5'000 | " | 3,7 | 60 | 100 | 99,7 | 88,1(S) |
| f | $C_6H_5O$ | " | 5'000 | " | 3,7 | 60 | 100 | 100,0 | 92,8(S) |
| g | $C_6F_5O$ | " | 5'000 | " | 3,7 | 60 | 100 | 64,2 | 91,7(S) |
| h | $CH_3COCH_2COCH_3$ | " | 5'000 | " | 3,7 | 60 | 100 | 98,9 | 91,8(S) |
| i | $CF_3COCH_2COCF_3$ | " | 5'000 | " | 3,7 | 60 | 100 | 96,2 | 93,7(S) |

Beispiel 3

In einer Glove-Box ($O_2$-Gehalt <1 ppm) wird in einem 50 ml Messkolben eine Katalysatorlösung durch Lösen von 15,9 mg (0,024 mMol) Di-μ-Trifluoracetat-bis-(1,5-cydooctadien)dirhodium und 33,2 mg (0,049 mMol) (R)-p-TolBINAP in 50 ml Toluol hergestellt. 5 ml dieser Katalysatorlösung werden zu einer Lösung von 5,0 g (24,47 mMol) (E)-Dehydroliliol in 145 ml Toluol in einen 500 ml Autoklav gegeben. Die Hydrierung wird bei 100°C, einem konstanten Druck von 60 bar und unter intensivem Rühren durchgeführt. Nach 21 Stunden beträgt der Umsatz 100%. Die hellgelbe Hydrierlösung wird wie in Beispiel 2 beschrieben aufgearbeitet. Man erhält (S)-Liliol mit einer enantiomeren Reinheit von 91,0% e.e.

Beispiel 4

In einer Glove-Box ($O_2$-Gehalt <1 ppm) wird in einem 100 ml Messkolben eine Katalysatorlösung durch Lösen von 19,9 mg (0,0493 mMol) Bis-(1,5-Cyclooctadien)rhodium(I)tetrafluoroborat, 33,2 mg (0,0493 mMol) (R)-pTolBINAP und 14,9 mg (0,0493 mMol) Tetrabutylammoniumnitrat in 100 ml Toluol hergestellt. 10 ml dieser Katalysatorlösung werden zu einer Lösung von 5,0 g (24,47 mMol) (E)-Dehydroliliol in 140 ml Toluol in einen 500 ml Autoklav gegeben. Die Hydrierung wird bei 100°C, einem konstanten Druck von 60 bar und unter intensivem Rühren durchgeführt. Nach 17 Stunden beträgt der Umsatz 99,8%. Die hellgelbe Hydrierlösung wird wie in Beispiel 2 beschrieben aufgearbeitet. Man erhält (S)-Liliol mit einer enantiomeren Reinheit von 94,6% e.e. $[\alpha]_{365}^{20}$ = -47,6° (EtOH, c = 1%).

Beispiele 4a-

In zu Beispiel 4 analoger Weise wird eine Katalysatorlösung hergestellt und anschliessend die Hydrierung von (E)-Dehydroliliol durchgeführt. Die Resultate sind in Tabelle 2 zusammengefasst:

Tabelle 2

| Beispiele 4a-4l | | | | | | | | | |
|---|---|---|---|---|---|---|---|---|---|
| No 4 | [Rh(X)(Y)(COD)] X | Y | S/C | Solvens | c % | p bar | T °C | % Umsatz nach 22 h | Liliol e.e. % |
| a | $CH_3COO$ | (R)-pTolBINAP | 10'000 | Toluol | 11,0 | 60 | 100 | 36,2 | 92,6(S) |
| b | F | " | 10'000 | " | 11,0 | 60 | 100 | 53 | 93,2(S) |
| c | Br | " | 10'000 | " | 11,0 | 60 | 100 | 96,8 | 96,2(S) |
| d | J | " | 10'000 | " | 11,0 | 60 | 100 | 87,9 | 95,8(S) |
| e | $NO_3$ | " | 5'000 | " | 3,7 | 60 | 100 | 99,8 | 94,6(S) |
| f | $NO_2$ | " | 5'000 | " | 3,7 | 60 | 100 | 98,7 | 91,7(S) |
| g | OH | " | 5'000 | " | 3,7 | 60 | 100 | 99,3 | 89,7(S) |
| h | CN | " | 5'000 | " | 3,7 | 60 | 100 | 97,9 | 95,5(S) |
| i | SCN | " | 5'000 | " | 3,7 | 60 | 100 | 94,6 | 95,4(S) |
| j | OCN | " | 5'000 | " | 3,7 | 60 | 100 | 98,5 | 93,7(S) |
| k | $HSO_4$ | " | 5'000 | " | 3,7 | 60 | 100 | 48,8 | 88,0(S) |
| l | $H_2PO_4$ | " | 5'000 | " | 3,7 | 60 | 100 | 99,0 | 88,2(S) |

Beispiel 5

In einer Glove-Box ($O_2$-Gehalt <1 ppm) wird in einem 50 ml Messkolben eine Katalysatorlösung durch Lösen von 24,3 mg (0,0493 mMol) Dichlor-bis(1,5-Cyclooctadien)dirhodium und 66,9 mg (0,0986 mMol) (R)-pTolBINAP in 50 ml Toluol hergestellt. 2 ml dieser Katalysatorlösung werden zu einer Lösung von 16,1 g (78,85 mMol) (E)-Dehydroliliol in 148 ml Toluol in einen 500 ml Autoklav gegeben. Die Hydrierung wird bei 100°C, einem konstanten Druck von 60 bar und unter intensivem Rühren durchgeführt. Nach 21 Stunden beträgt der Umsatz 99,6%. Die hellgelbe Hydrierlösung wird wie in Beispiel 2 beschrieben aufgearbeitet. Man erhält (S)-Liliol mit einer enantiomeren Reinheit von 95,1 % e.e. $[\alpha]^{20}_{365}$ = -47,0° (EtOH, c = 1%).

Beispiele 5a-

In zu Beispiel 5 analoger Weise wird die Katalysatorlösung hergestellt und die Hydrierung unter den in Tabelle 3 angegebenen Bedingungen durchgeführt. Die Hydrierlösung wird wie in Beispiel 2 aufgearbeitet. Die Resultate sind in

Tabelle 3 zusammengefasst:

Tabelle 3

| Beispiele 5a-5j | | | | | | | | | |
|---|---|---|---|---|---|---|---|---|---|
| No 5 | [Rh(X)(Y)(COD)] X | Y | S/C | Solvens | c % | p bar | T °C | % Umsatz nach 22 h | Liliol e.e. % |
| a | Cl | (R)-BIPHEMP | 200 | Toluol | 11 | 60 | 20 | 100,0 | 93,0(S) |
| b | Cl | " | 200 | " | 11 | 60 | 60 | 100,0 | 93,7(S) |
| c | Cl | (R)-pTolBINAP | 20'000 | " | 11 | 60 | 120 | 99,0 | 91,0(S) |
| d | Cl | " | 20'000 | " | 11 | 30 | 100 | 90,5 | 96,4(S) |
| e | Cl | " | 20'000 | " | 11 | 5 | 100 | 25,6 | 94,6(S) |
| f | Cl | " | 20'000 | " | 30 | 60 | 100 | 53,0 | 90,9(S) |
| g | Cl | (R)-pTolMeOBIPHEP | 20'000 | " | 11 | 60 | 100 | 100 | 94,2(S) |
| h | Cl | (R)-BIPHOMP | 20'000 | " | 11 | 60 | 100 | 100 | 92,6(S) |
| i | Cl | (R)-BIPHEMP | 200 | THF | 11 | 60 | 60 | 100 | 93,7(S) |
| j | Cl | (R)-pTolBINAP | 10'000 | AcOEt | 11 | 60 | 100 | 99 | 95,2(S) |

Beispiel 6

In einer Glove-Box ($O_2$-Gehalt <1 ppm) wird in einem 50 ml Messkolben eine Katalysatorlösung durch Lösen von 32,0 mg (0,079 mMol) Bis-(1,5-Cyclooctadien)rhodium(I)tetrafluoroborat und 53,5 mg (0,079 mMol) (R)-pTolBINAP in 50 ml Toluol hergestellt. 5 ml dieser Katalysatorlösung werden zu einer Lösung von 16,1 g (78,85 mMol) (E)-Dehydroliliol in 145 ml Toluol in einen 500 ml Autoklav gegeben. Die Hydrierung wird bei 100°C, einem konstanten Druck von 60 bar und unter intensivem Rühren durchgeführt. Nach 22 Stunden beträgt der Umsatz 99,8%. Die hellgelbe Hydrierlösung wird wie in Beispiel 2 beschrieben aufgearbeitet. Man erhält (S)-Liliol mit einer enantiomeren Reinheit von 95,4% e.e. $[\alpha]^{20}_{365}$ = -47,5 (EtOH, c = 1%).

Beispiel 7

In einer Glove-Box ($O_2$-Gehalt <1 ppm) wird eine Katalysatorlösung durch Lösen von 9,15 mg (0,0186 mMol) Dichlor-bis-(1,5-Cyclooctadien)dirhodium und 25,18 mg (0,0371 mMol) (R)-pTolBINAP in 50 ml Toluol hergestellt. Diese Katalysatorlösung wird zu einer Lösung von 5,5 g (37,1 mMol) (E)-2-Methyl-3-phenyl-2-propen-1-ol in 35 ml Toluol in einen 500 ml Autoklav gegeben. Die Hydrierung wird bei 100°C, einem konstanten Druck von 60 bar und unter intensivem Rühren durchgeführt. Nach 22 Stunden beträgt der Umsatz 100%. Die hellgelbe Hydrierlösung wird wie in Beispiel 2 beschrieben aufgearbeitet. Man erhalt (S)-2-Methyl-3-phenylpropan-1-ol mit einer enantiomeren Reinheit von 89,1% e.e., $[\alpha]^{20}_{365}$ = -57,9° (EtOH, c = 1%).

Beispiel 8

(a) (S)-1-tert.Butyl-4-[3-chlor-2-methylpropyl]-benzol

30,9 g (0,15 Mol) (S)-3-(p-tert.Butylphenyl)--2-methyl-1-propanol (S-Liliol) werden vorgelegt und bei 120°C innerhalb von 5 Stunden unter Rühren mit 20,2 g (0,17 Mol) Thionylchlorid versetzt. Die dabei entstehenden gasförmigen Produkte ($SO_2$, HCl) werden zur Vernichtung in einen mit 10%iger Natronlauge gefüllten Waschturm geleitet. Nach beendetem Zutropfen wird noch eine halbe Stunde bei 120°C nachgerührt und dann abgekühlt. Das Rohprodukt wird am Hochvakuum (Sdp. ca. 80°C) destilliert. Man erhält ein farbloses Oel von (a).

(b) (S)-2-Methyl-3-phenyl-propylchlorid

In analoger Weise wie unter (a) beschrieben werden 22,5 g (0,15 Mol) (S)-2-Methyl-3-phenyl-propanol mit 20,2 g (0,17 Mol) Thionylchlorid umgesetzt und das Rohprodukt am Wasserstrahlvakuum (18 Torr) zu (b) destilliert (Sdp. 110-112°C); farbloses Oel.

Das Produkt (b) kann nun auf bekannte Weise durch eine Friedel-Crafts-Alkylierung z.B. mit Isobutylen in konz. Schwefelsäure in das Produkt (a) umgewandelt werden.

Die Umsetzung von (a) mit Piperidin führt zur neuen Verbindung (S)-Fenpropidin.

Die Umsetzung von (a) mit cis-2,6-Dimethyl-morpholin führt zum vorbekannten (S)-Fenpropimorph.

<u>(S)-1-[3-(p-tert.Butylphenyl)-2-methylpropyl]-piperidin ((S)-Fenpropidin)</u>

27 g (0,12 Mol) (S)-1-tert.Butyl-4-[3-chlor-2-methylpropyl]-benzol werden in 70 ml (0,708 Mol) Piperidin 16 Stunden unter intensivem Rühren auf Rückflusstemperatur erhitzt. Anschliessend giesst man weitere 30 ml (0,303 Mol) Piperidin zu. Der Reaktionsverlauf wird gaschromatographisch verfolgt. Man rührt das Reaktionsgemisch eine weitere Stunde bei Rückflusstemperatur und lässt es abkühlen. Man giesst auf 350 ml Natronlauge und extrahiert mit drei Portionen, insgesamt 600 ml, n-Hexan. Die organischen Phasen werden vereinigt, über wasserfreiem Natriumsulfat getrocknet und eingeengt. Man chromatographiert den Rückstand an 700 g Kieselgel 60 (MERCK, Korngrösse, 0,040-0,063 mm) mit Essigsäureäthylester/n-Hexan (1:1). Die reinen Fraktionen werden vereinigt und unter Hochvakuum (Sdp. ca. 140°C) destilliert. Man erhält ein farbloses Oel. Opt. Drehung (c=1%, $C_2H_5OH$ 96%):
365 nm +11,1°
436 nm + 9,0°
546 nm + 6,0°
578 nm + 5,5°
589 nm + 5,4°

Unter Zugabe des "chiralen Lösungsmittels" (R)-(-)-2,2,2-Trifluor-1-(9-anthryl)-äthanol (TAE) lässt sich im NMR-Spektrum ($CDCl_3$) die enantiomere Reinheit bestimmen. Die Verschiebungen der Signale der Methylgruppe am Chiralitätszentrum ist von der relativen Menge von TAE in der Lösung abhängig: Es wird kein Hinweis auf Racemisierung gefunden.

Die Umsetzung von (a) mit cis-3,5-Dimethylpiperidin führt zum neuen Verbindung cis-1-[(S)-3-(p-tert.Butylphenyl)-2-methylpropyl]-3,5-dimethylpiperidin, farbloses Oel. Optische Drehung in 1% EtOH: $[\alpha]_{546} = +6,1°$.

Die Umsetzung von (a) mit rac.-3-Methylpiperidin führt zum neuen Fungicid (RS)-1-[(S)-3-(p-tert.Butylphenyl)-2-methylpropyl]-3-methylpiperidin (Epimerenverhältnis 1:1); farbloses Oel.

<u>Beispiel 9</u>

Ausgehend von dem in Beispiel 7 hergestellten (S)-2-Methyl-3-phenylpropan-1-ol werden entsprechend dem obigen Reaktionsschema die folgenden neuen, fungicid aktiven Verbindungen hergestellt:

(S)-1-[3-(p-tert.Amylphenyl)-2-methylpropyl]-piperidin, als farbloses Oel;
cis-1-[(S)-3-(p-tert.Amylphenyl)-2-methylpropyl]-3,5-dimethylpiperidin, als farbloses Oel;
(RS)-1-[(S)-3-(p-tert.Amylphenyl)-2-methylpropyl]-3-methylpiperidin, als farbloses Oel.

**Patentansprüche**

**1.** Verfahren zur Herstellung der Verbindungen der Formel

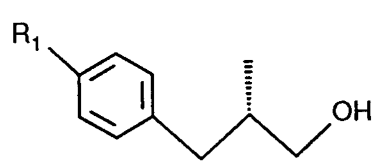

I

worin $R_1$ Wasserstoff oder eine Gruppe Z,

Z die Gruppe

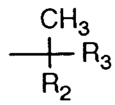 ,

$R_2$ Methyl, Ethyl, Chlormethyl und
$R_3$ $C_1$-$C_4$-Alkyl bedeuten,
oder $R_2$ und $R_3$ zusammen mit dem Kohlenstoffatom, an das sie gemeinsam gebunden sind, $C_3$-$C_7$-Cycloalkyl darstellen, durch asymmetrische Hydrierung einer Verbindung der Formel

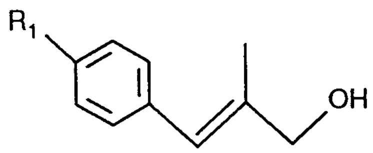

II

in der E-Form,
worin $R_1$ obige Bedeutung hat, dadurch gekennzeichnet, dass man die Hydrierung in Gegenwart eines Rhodiumkatalysators der Formeln V oder VI durchführt

$$[Rh(X)(Y)L_{0,1,2}]_{1,2} \qquad V$$

oder

$$[Rh(Y)L_{0,1,2}]^{+}A^{-} \qquad VI$$

worin bedeuten:
X ein koordinierender anionischer Ligand, wie Halogen, ein Carbonsäurerest, ein 1,3-Diketonat, ein (gegebenenfalls substituiertes) Phenolat, Hydroxy, Nitrat, Nitrit, Cyanat, Rhodanid, Cyanid, Hydrogensulfat;
Y ein chiraler atropisomerer Diphosphinligand, der Formeln VII oder VIII:

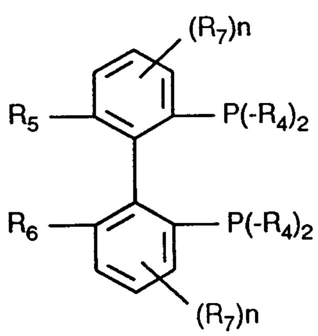

VII

worin $R_4$ Aryl, Cyclohexyl, $R_5$ und $R_6$ Wasserstoff, niederes Alkyl, niederes Alkoxy, Di-niederes Alkylamino, geschütztes Hydroxymethyl oder $R_5$ und $R_6$ zusammen die Gruppen
$(-CH_2)-)_m$, $-CH_2-O-CH_2-$, $-CH_2-NR_8-CH_2-$ oder $-CH_2-C(OR_9)_2-CH_2-$
bedeuten, wobei m eine Zahl 3 bis 5, $R_8$ niederes Alkyl, Aryl oder Benzyl und $R_9$ niederes Alkyl oder beide $R_9$ zusammen Di- oder Trimethylen darstellen, $R_7$ Methyl, niederes Alkoxy, Di-niederes Alkylamino oder Halogen und

n die Zahl 0, 1, 2 oder 3 bedeuten;

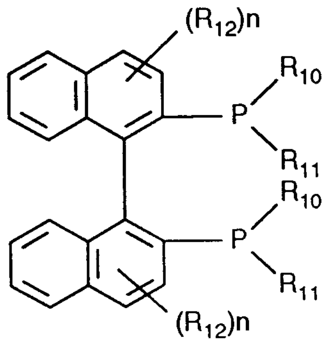

VIII

worin $R_{10}$ und $R_{11}$ Aryl und Cyclohexyl, $R_{12}$ Methyl, Ethyl, Halogen, -OH; $NH_2$, Acetylamino, Nitro, $-SO_3H$, bevorzugt in der 5,5'-Position,
L ein neutraler Ligand,
A- ein Anion, insbesondere $BF_4$- bedeuten.

2. Verfahren gemäss Anspruch 1, dadurch gekennzeichnet, dass man die asymmetrische Hydrierung bei einer Temperatur von 20°C bis 140°C, vorzugsweise von 80°C bis 120°C durchführt.

3. Verfahren gemäss Anspruch 1, worin der Diphosphinligand der Formel VII
R-(6,6'-Dimethyl-2,2'-biphenylylen)bis(diphenylphosphin);
R-(6,6'-Dimethyl-2,2'-biphenylylen)bis(di-p-tolylphosphin);
R-(6,6'-Dimethoxy-2,2'-biphenylylen)bis(diphenylphosphin); oder
R-(6,6'-Dimethoxy-2,2'-biphenylylen)bis(di-p-tolylphosphin)
ist.

## Claims

1. A process for the preparation of a compound of formula

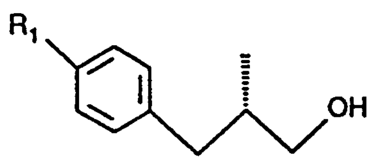

I

wherein $R_1$ is hydrogen or a group Z, Z is the group

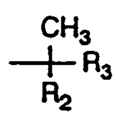

,

$R_2$ is methyl, ethyl or chloromethyl and $R_3$ is $C_1$-$C_4$alkyl, or $R_2$ and $R_3$, together with the carbon atom to which they are jointly attached, are $C_3$-$C_7$cycloalkyl, by asymmetrically hydrogenating a compound of formula

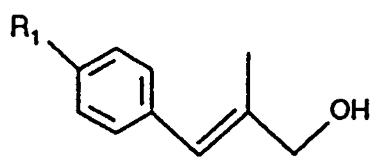

II

in the E form, wherein $R_1$ is as defined above, which comprises carrying out the hydrogenation in the presence of a rhodium catalyst of formula V or VI

$$[Rh(X)(Y)L_{0,1,2}]_{1,2} \qquad V$$

or

$$[Rh(Y)L_{0,1,2}]^{+}A^{-} \qquad VI$$

wherein
X is a co-ordinating anionic ligand, such as halogen, a carboxylic acid radical, a 1,3-diketonate, an (unsubstituted or substituted) phenolate, hydroxy, nitrate, nitrite, cyanate, thiocyanate, cyanide, hydrogensulfate;
Y is a chiral atropisomeric diphosphine ligand of formula VII or VIII;

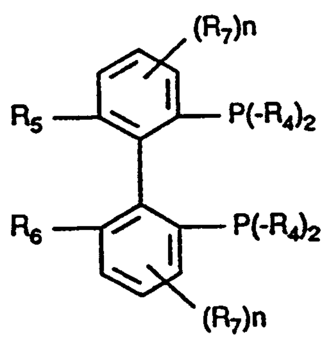

VII

wherein $R_4$ is aryl or cyclohexyl, $R_5$ and $R_6$ are hydrogen, lower alkyl, lower alkoxy, di-lower alkylamino or protected hydroxymethyl, or $R_5$ and $R_6$ together are a group
$(-CH_2-)_m$, $-CH_2-O-CH_2-$, $-CH_2-NR_8-CH_2-$ or $-CH_2-C(OR_9)_2-CH_2-$,
where m is a number from 3 to 5, $R_8$ is lower alkyl, aryl or benzyl, and $R_9$ is lower alkyl, or both substituents $R_9$ together are di- or trimethylene, $R_7$ is methyl, lower alkoxy, di-lower alkylamino or halogen, and n is 0, 1, 2 or 3;

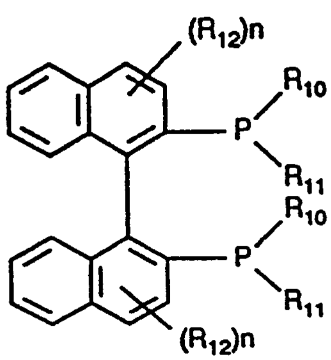

VIII

wherein $R_{10}$ and $R_{11}$ are aryl or cyclohexyl, and $R_{12}$ is methyl, ethyl, halogen, -OH; $NH_2$, acetylamino, nitro, $-SO_3H$, preferably in 5,5'-position,
L is a neutral ligand, and
$A^-$ is an anion, preferably $BF_4^-$.

**2.** A process according to claim 1, wherein the asymmetrical hydrogenation is carried out at a temperature from 20°C to 140°C, preferably from 80°C to 120°C.

**3.** A process according to claim 1, wherein the diphosphine ligand of formula VII is
R-(6,6'-dimethyl-2,2'-biphenylene)bis(diphenylphosphine);
R-(6,6'-dimethyl-2,2'-biphenylene)bis(di-p-tolylphosphine);

R-(6,6'-dimethoxy-2,2'-biphenylene)bis(diphenylphosphine); or
R-(6,6'-dimethoxy-2,2'-biphenylene)bis(di-p-tolylphosphine).

**Revendications**

1. Procédé de préparation des composés de formule

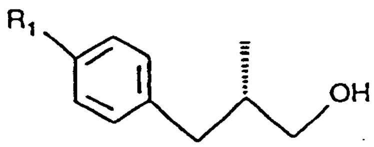

I

dans laquelle
$R_1$ représente l'hydrogène ou un groupe Z,
Z représente le groupe

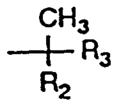

,

$R_2$ représente un groupe méthyle, éthyle, chlorométhyle et
$R_3$ représente un groupe alkyle en $C_1$-$C_4$,
ou bien $R_2$ et $R_3$ forment ensemble et avec l'atome de carbone auquel ils sont reliés un groupe cycloalkyle en $C_3$-$C_7$,
par hydrogénation asymétrique d'un composé de formule

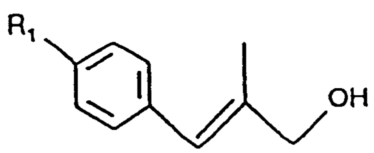

II

sous la forme E,
$R_1$ ayant les significations indiquées ci-dessus, ce procédé se caractérisant en ce que l'on réalise l'hydrogénation en présence d'un catalyseur au rhodium de formule V ou VI

$$[Rh(X)(Y)L_{0,1,2}]_{1,2} \quad V$$

ou

$$[Rh(Y)L_{0,1,2}]^{+}A^{-} \quad VI$$

dans lesquelles :
X représente un ligand anionique coordinant tel qu'un halogène, un radical d'acide carboxylique, un 1,3-dicétonate, un phénolate (éventuellement substitué), un groupe hydroxy, un nitrate, un nitrite, un cyanate, un thiocyanate, un cyanure, un groupe bisulfate ;

Y représente un ligand diphosphinique chiral atropisomère de formule VII ou VIII

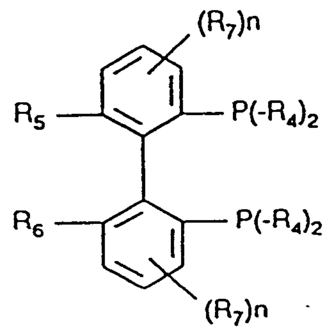

VII

dans laquelle $R_4$ représente un groupe aryle, cyclohexyle, $R_5$ et $R_6$ représentent l'hydrogène, des groupes alkyle inférieur, alcoxy inférieur, di-(alkyle inférieur)amino, hydroxyméthyle protégé ou bien $R_5$ et $R_6$ forment ensemble les groupes

$(-CH_2)-)_m$, $-CH_2-O-CH_2-$, $-CH_2-NR_8-CH_2-$ ou $-CH_2-C(OR_9)_2-CH_2$,

dans lesquels m est un nombre allant de 3 à 5, $R_8$ représente un groupe alkyle inférieur, aryle ou benzyle et $R_9$ un groupe alkyle inférieur, ou bien deux groupes $R_9$ forment ensemble un groupe di- ou tri-méthylène, $R_7$ représente un groupe méthyle, alcoxy inférieur, di-(alkyle inférieur)-amino ou un halogène et n est égal à 0, 1, 2 ou 3 ;

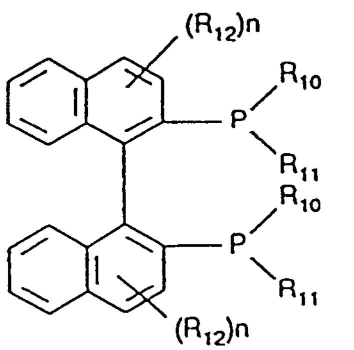

VIII

dans laquelle
$R_{10}$ et $R_{11}$ représentent des groupes aryle et cyclohexyle,
$R_{12}$ un groupe méthyle, éthyle, un halogène, un groupe -OH ; un groupe $NH_2$, acétylamino, nitro, $-SO_3H$, de préférence en position 5,5',
L représente un ligand neutre,
A représente un anion, plus spécialement l'anion $BF_4^-$.

2. Procédé selon revendication 1, caractérisé en ce que l'hydrogénation asymétrique est réalisée à des températures de 20 à 140°C, de préférence de 80 à 120°C.

3. Procédé selon revendication 1, dans lequel le ligand diphosphinique de formule VII est :
   la R-(6,6'-diméthyl-2,2'-biphénylylène)-bis-(diphénylphosphine);
   la R-(6,6'-diméthyl-2,2'-biphénylylène)-bis-(di-p-tolylphosphine);
   la R-(6,6'-diméthoxy-2,2'-biphénylylène)-bis-(diphénylphosphine); ou bien
   la R-(6,6'-diméthoxy-2,2'-biphénylylène)-bis-(di-p-tolylphosphine).